# DEMANDE DE BREVET EUROPEEN

(11) **EP 0 970 716 A1**
(43) Date de publication de la demande: **12.01.2000**
(21) Numéro de dépôt: 99401461.1
(22) Date de dépôt: 15.06.1999
(51) Int. Cl.: A61M 27/00

(54) **Dispositifs pour le drainage de fluides biologiques**

(30) Priorité: 10.07.1998 FR 9808870
(71) Demandeur: NMT Neurosciences Implants S.A., 06921 Sophia Antipolis (FR)
(72) Inventeur: Lecuyer, Alain, 06130 Grasse (FR)
(74) Mandataire: Geismar, Thierry

(57) **Abrégé**

L'invention concerne des dispositifs pour le drainage de fluides biologiques.

Un premier dispositif comprend un boîtier (2) et une vanne à pincement (3) montée dans le boîtier à l'écart des parois de ce dernier. Le boîtier comporte une ouverture (11) en vis à vis des moyens de pincement (6) de la vanne. Un autre dispositif (13) comprend un tronçon de cathéter (7) de ligne de drainage et un organe (12) de support de cathéter, le tronçon de cathéter pénétrant dans et sortant de l'organe de support (12) en deux points et faisant une boucle entre ces deux points.

Application au drainage ventriculaire externe.

## Description

La présente invention concerne des dispositifs pour le drainage de fluides biologiques.

Des dispositifs de ce type sont bien connus dans l'état de la technique. Plus particulièrement, la demande de brevet français n° 9716020 décrit un dispositif de drainage ventriculaire externe comprenant un cathéter dont une extrémité est disposée dans la zone à drainer et l'autre extrémité est reliée à un sac de drainage.

Ce dispositif comprend en outre un capteur de pression disposé sur le cathéter à proximité de son extrémité située du côté de la tête du patient et une valve extérieure à pincement sur ce cathéter. Une alimentation à commande électronique est prévue pour ouvrir la valve lorsque la pression dépasse un seuil prédéterminé. La valve à pincement est, bien entendu, réutilisable alors que le cathéter est remplacé à chaque utilisation.

Un problème rencontré avec ce type de dispositif réside dans la difficulté de mise en place du cathéter dans la vanne à pincement. Cette mise en place doit être effectuée en effet avec un soin tout particulier si l'on souhaite que le dispositif fonctionne convenablement.

Par ailleurs, le cathéter utilisé doit être compatible avec la vanne. En particulier, son diamètre et la souplesse du matériau dans lequel il est réalisé doivent être adaptés. Une erreur dans le choix du cathéter utilisé peut conduire à un dysfonctionnement du dispositif.

Enfin, les vannes à pincement, tout en étant des dispositifs robustes, nécessitent néanmoins certaines précautions lors de leur utilisation et de leur stockage. Il est donc préférable que ces vannes et notamment leur zone de pincement soient protégées par un boîtier.

La présente invention vise à pallier ces inconvénients.

Plus particulièrement, l'invention vise à fournir des dispositifs utilisables pour le drainage de fluides biologiques à l'aide d'un cathéter et des moyens de pincement de ce cathéter permettant d'assurer que ce dernier est parfaitement adapté aux moyens de pincement. L'invention vise également à fournir des moyens permettant un engagement convenable du cathéter dans ces moyens de pincement. L'invention vise également encore à fournir des moyens de drainage de fluide biologique dont les moyens de pincement sont protégés.

A cet effet, l'invention a tout d'abord pour objet un dispositif pour le drainage de fluides biologiques, caractérisé par le fait qu'il comprend un boîtier muni de parois et une vanne à pincement comprenant des moyens de pincement, ladite vanne étant montée dans le boîtier à l'écart desdites parois, ledit boîtier comportant une ouverture en vis à vis desdits moyens de pincement, des moyens étant par ailleurs prévus pour engager un tronçon de cathéter de ligne de drainage dans lesdits moyens de pincement à travers ladite ouverture.

L'invention a également pour objet un dispositif pour le drainage de fluides biologiques, caractérisé par le fait qu'il comprend un tronçon de cathéter de ligne de drainage et un organe de support de cathéter, ledit tronçon de cathéter pénétrant dans et sortant dudit organe de support en deux points et faisant une boucle entre lesdits deux points, ledit support de cathéter présentant une découpe laissant libre une partie de ladite boucle pour permettre son pincement par des moyens de pincement.

On comprend que les deux dispositifs qui viennent d'être décrits sont agencés pour coopérer, même s'ils ne sont physiquement réunis que lors de leur utilisation. En particulier, le support de cathéter présente une forme telle qu'il peut être introduit dans l'ouverture du boîtier de façon à amener la partie du cathéter laissée libre par la découpe dans les moyens de pincement de la vanne montée à l'intérieur de ce boîtier.

L'invention permet de pallier les inconvénients mentionnés ci-dessus. En effet, en premier lieu, le support de cathéter peut être guidé lors de sa pénétration dans le boîtier par tout moyen convenable de manière que la partie libre de la boucle du tube s'engage parfaitement dans les moyens de pincement de la vanne.

Par ailleurs, la vanne étant disposée dans le boîtier à l'écart des parois de ce dernier, le tronçon de cathéter de la ligne de drainage doit impérativement être amené dans les moyens de pincement à travers l'ouverture du boîtier. On peut donc faire en sorte, en dimensionnant convenablement cette ouverture, qu'une mise en place manuelle du cathéter ne soit pas matériellement possible.

Dans ces conditions, on est également sûr qu'un cathéter présentant les caractéristiques convenables est utilisé. Il suffit en effet de rendre indémontable le tronçon de cathéter utilisé et son organe de support et d'introduction.

Enfin, la vanne à pincement et tout particulièrement ces moyens de pincement sont protégés par le boîtier.

Dans un mode de réalisation particulier de l'invention, ledit support de cathéter comprend des moyens élastiques pour son blocage par rapport auxdits moyens de pincement.

Ces moyens élastiques peuvent en particulier coopérer avec la paroi du boîtier dans laquelle est formée son ouverture.

Egalement dans un mode de réalisation particulier, ledit support de cathéter forme une cassette plate agencée pour être introduite dans une fente desdits moyens de pincement.

Dans ce cas, l'ouverture précitée ménagée dans le boîtier est donc en forme de fente, forme correspondant à la section transversale de la cassette.

Plus particulièrement, ladite cassette peut être en forme de T, ledit cathéter pénétrant dans et sortant de la cassette aux extrémités de la barre horizontale du T, et ladite boucle étant formée dans la branche verticale du T.

Dans ce cas, les dispositifs peuvent être agencés de sorte qu'en utilisation, seule la barre horizontale du T de la cassette fasse saillie de la fente du boîtier, cette barre formant ainsi un organe de préhension pour le retrait de la cassette de la fente.

On peut également prévoir que ladite boucle forme une partie de cathéter séparée d'une partie d'entrée et d'une partie de sortie, raccordées dans ledit support de cathéter auxdites parties d'entrée et de sortie.

Il est ainsi possible de réaliser la boucle du cathéter dans un matériau particulièrement adapté à son utilisation dans une vanne à pincement, le reste du cathéter présentant des caractéristiques différentes.

On décrira maintenant, à titre d'exemple non limitatif, un mode de réalisation particulier de l'invention, en référence aux dessins schématiques annexés dans lesquels :
- la figure 1a est une vue en perspective d'un des dispositifs objet de l'invention;
- la figure 1b est une vue également en perspective et partiellement arrachée de l'autre dispositif de l'invention;
- la figure 2 est une vue en coupe schématique d'une vanne à pincement susceptible d'être utilisée dans l'invention;
- la figure 3 est une vue en plan d'un des dispositifs objet de l'invention avant son montage;
- la figure 4 est une vue de dessus de ce dispositif, en cours de montage; et
- la figure 5 est une vue en coupe d'une partie de ce dispositif;

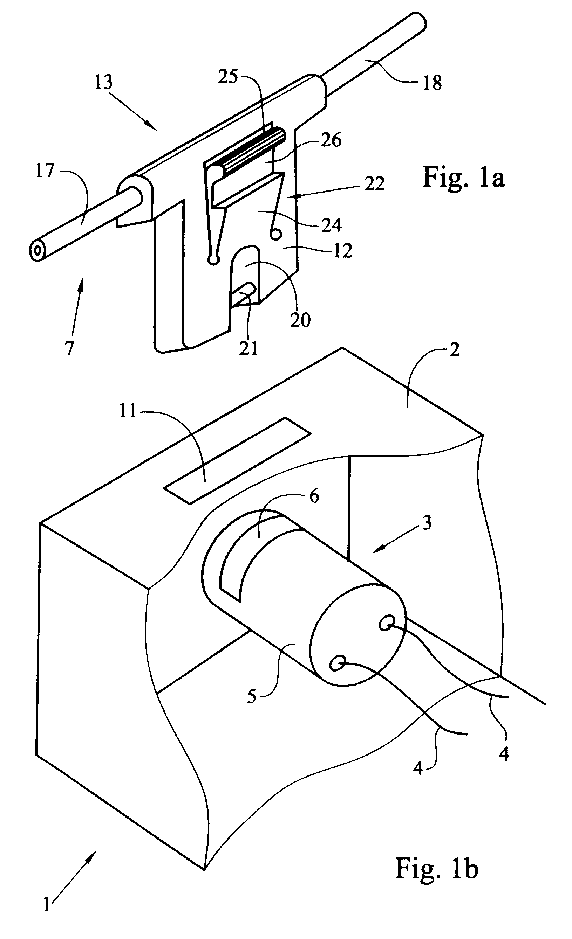

On voit à la figure 1b un dispositif 1 de drainage de fluide biologique comportant pour l'essentiel un boîtier 2 dans lequel une électrovanne à pincement 3 est montée de toute manière convenable à l'écart des parois du boîtier 2. Des fils d'alimentation électriques 4 pour l'électrovanne 3 sortent du boîtier 2.

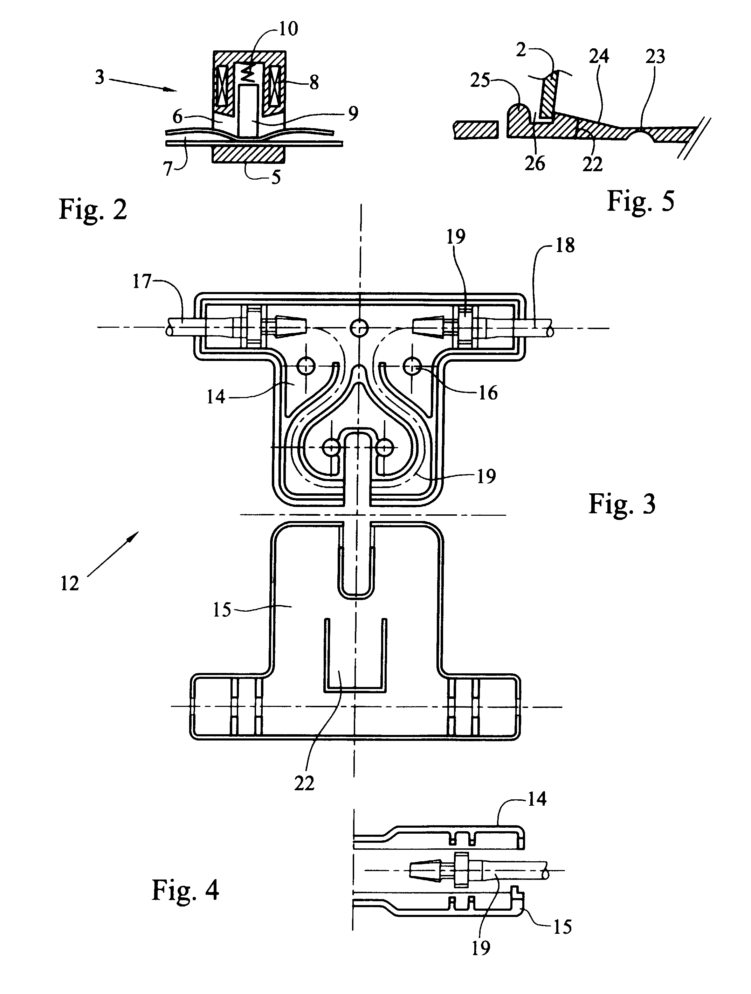

L'électrovanne 3 est représentée plus en détails à la figure 2.

Cette électrovanne est composée d'un corps 5 formant un étrier ménageant une fente 6 dans laquelle un cathéter 7 peut être engagé pour être fermé par pincement.

Une des branches du corps 5 reçoit un électroaimant dont le bobinage 8 est alimenté à partir des fils 4. Le noyau 9 de cet électroaimant forme un plongeur susceptible de pincer le cathéter 7 contre l'autre branche du corps 5. Un ressort de compression 10 est disposé entre le corps 5 et le noyau 9 pour pousser le noyau en direction du cathéter 7 et le pincer.

En revenant à la figure 2, on voit que la paroi supérieure du boîtier 2 présente une ouverture 11 en forme de fente située directement en vis-à-vis de la fente 6 de l'électrovanne à pincement 3.

Comme montré à la figure 1a, le cathéter 7 est ici solidaire d'une cassette 12, formant avec le cathéter 7 l'autre dispositif 13 objet de l'invention.

La cassette 12 est réalisée en matière plastique moulée, à partir de deux demi-cassettes 14 et 15, réunies entre elles pour former un espace de logement de cathéter et collée à l'aide de plots de collage 16.

La cassette 12 est en forme générale de T. Une partie 17 d'entrée de cathéter pénètre dans la cassette 12 à une extrémité de la barre horizontale du T et une partie 18 de sortie de cathéter sort de la cassette 12 à l'autre extrémité de cette barre. Entre ces deux parties d'entrée et de sortie de cathéter, une partie de boucle représentée à la figure 3 uniquement par son axe 19 est logée à l'intérieur de la barre verticale du T. Des raccords 19 servent à raccorder les différentes parties 17, 19 et 18 du cathéter 7.

La branche verticale du T formant la cassette 12 présente une section transversale de même forme que la fente 11, de manière à pouvoir être glissée dans cette fente. Par ailleurs, cette branche comporte une découpe 20, laissant libre une partie 21 de la boucle du cathéter.

Lorsque la cassette 12 est engagée dans la fente 11 avec la barre horizontale du T dépassant de cette fente, la partie libre 21 du cathéter est engagée dans la fente 6 entre le boîtier 5 et le plongeur 9.

Enfin, la partie 15 de la cassette 12 comporte une languette élastique 22 à laquelle elle est reliée par un pont de matière 23 formant charnière. La languette 22 présente une surface inclinée 24, séparée d'une partie 25 formant poussoir par une encoche 26.

Lorsque la cassette 12 est engagée dans la fente 11, le bord de cette dernière coopère avec la rampe 24 pour faire fléchir la languette 22 vers l'intérieur de la cassette. Lorsque la cassette 12 arrive au bout de sa course, la paroi du boîtier 2 s'enclenche dans l'encoche 26 et verrouille ainsi la cassette 12, la rendant solidaire du boîtier 2.

Ce n'est qu'en appuyant sur le poussoir 25 et en saisissant la partie de la cassette 12 qui dépasse de la fente 11 que l'on peut déverrouiller cette cassette et l'extraire du boîtier. Toute extraction involontaire est ainsi rendue impossible.

## Revendications

1. Dispositif (1) pour le drainage de fluide biologique caractérisé par le fait qu'il comprend un boîtier (2) muni de parois, une vanne à pincement (3) comprenant des moyens de pincement (8, 9), ladite vanne étant montée dans le boîtier à l'écart desdites parois, ledit boîtier comportant une ouverture (11) en vis-à-vis des moyens de pincement, des moyens (12) étant par ailleurs prévus pour engager un tronçon de cathéter (7) de ligne de drainage dans lesdits moyens de pincement à travers ladite ouverture (11).

2. Dispositif (13) pour le drainage de fluide biologique, caractérisé par le fait qu'il comprend un tronçon de cathéter (7) de ligne de drainage et un organe (12) de support de cathéter, ledit tronçon de cathéter (7) pénétrant dans et sortant dudit organe (12) de support en deux points et faisant une boucle (19) entre lesdits deux points, ledit organe de support de cathéter présentant une découpe (21) laissant libre une partie (22) de ladite boucle pour permettre son pincement par des moyens de pincement.

3. Dispositif selon la revendication 2, dans lequel ledit organe (12) de support de cathéter comprend des moyens élastiques (23) pour son blocage par rapport auxdits moyens de pincement.

4. Dispositif selon l'une quelconque des revendications 2 et 3, dans lequel ledit organe (12) de support de cathéter forme une cassette plate, agencée pour être introduite dans une fente (11) desdits moyens de pincement.

5. Dispositif selon la revendication 4, dans lequel ladite cassette est en forme de T, ledit cathéter pénétrant dans et sortant de la cassette aux extrémités de la barre horizontale du T, et formant ladite boucle dans la branche verticale du T.

6. Dispositif selon l'une quelconque des revendications 2 à 5, dans lequel ladite boucle forme une partie de cathéter séparée d'une partie d'entrée (17) et d'une partie de sortie (18) raccordées dans ledit support de cathéter auxdites parties d'entrée et de sortie.
